# EUROPEAN PATENT SPECIFICATION

(11) **EP 2 992 927 B1**
(45) Date of publication and mention of the grant of the patent: **05.04.2017**
(21) Application number: 15178600.1
(22) Date of filing: 28.07.2015
(51) Int. Cl.: A61N 1/375, A61B 5/145, A61N 1/372, A61B 5/00, A61N 1/365, A61B 5/1473

(54) **ACTIVITY MONITOR AND RATE-ADAPTIVE IMPLANTABLE LEADLESS PACEMAKER**
AKTIVITÄTSÜBERWACHUNG UND RATENADAPTIVER IMPLANTIERBARER LEITUNGSLOSER SCHRITTMACHER
MONITEUR D'ACTIVITÉ ET STIMULATEUR CARDIAQUE SANS FIL IMPLANTABLE À FRÉQUENCE ASSERVIE

(30) Priority: 04.09.2014 US 201462045568 P
(43) Date of publication of application: 09.03.2016
(73) Proprietor: BIOTRONIK SE & Co. KG, 12359 Berlin (DE)
(72) Inventor: Baru, Marcelo, Tualatin, OR 97062 (US); Dabney, Warren, Lake Oswego, OR 97035 (US); Moulder, Christopher, Portland, OR 97202 (US)
(74) Representative: Galander, Marcus

(56) References cited:
- EP-A1- 1 503 661
- US-A1- 2008 262 322
- US-A1- 2009 131 738
- US-A1- 2012 109 236

## Description

The present invention generally relates to an activity monitor and to a rate-adaptive implantable leadless pacemaker comprising an activity monitor.

Leadless cardiac pacemakers as disclosed for example in US patent application US 2012/0109236 A1, are small devices implanted directly in a ventricle of a heart. Leadless cardiac pacemakers are preferably rate-adaptive to change pacing output dependent on patient activity. The problem is that sensing patient activity normally requires large circuitry with high power consumption for the application.

Leadless cardiac pacemakers disclosed in prior art adapt pacing rate based either on an activity sensor (accelerometer) or temperature sensor, and associated circuitry, hermetically contained within the housing.

Leadless pacemaker design has strict size and power consumption requirements. A motion-sensing activity sensor (e.g. an accelerometer) contained within the housing to adapt the pacing rate occupies substantial space and its associated circuitry (for signal processing) requires a power consumption level that reduces the lifetime of the device. A temperature sensor-based pacemaker, on the other hand, has a slow response time, which is detrimental to useful pacing rate adaptation.

In view of the above, there is a need for an improved rate-adaptive implantable leadless pacemaker.

European Patent EP 1 503 661 B1 discloses an activity monitor for monitoring metabolic demand that has two electrodes that are made of biocompatible materials that have different sensitivities to pH changes. The two electrodes are connected to an activity monitoring unit that is adapted to determine an open-circuit potential difference between said two electrodes and to generate an activity signal derived from said open-circuit potential difference.

It is an object of the invention to provide an improved leadless pacemaker and an activity monitor for monitoring metabolic demand.

The object of the invention is achieved by an implantable leadless pacemaker having a housing and at least two electrodes arranged on said housing. The two electrodes are made of biocompatible materials that have different sensitivities to pH changes. The two electrodes are connected to an activity monitoring unit that is adapted to periodically determine an open-circuit potential difference between the two electrodes and to generate an activity signal derived from said open-circuit potential difference. The implantable leadless pacemaker further comprises a control unit that is connected to the activity sensor and that is adapted to adjust a pacing rate in response to said activity signal generated by said activity monitoring unit. The implantable leadless pacemaker is characterized in that the activity monitoring unit or the control unit or both are configured to generate an activity signal indicating an increase of pacing rate or increasing the pacing rate, respectively, only when a sudden decrease of pH is determined.

Thus, the object of the invention is achieved by a leadless pacemaker with an activity monitor that utilizes the electrical properties of its electrodes in contact with blood to adapt the pacing rate.

Changes in metabolic needs, such as those caused by physical activities or emotional stress, will vary the venous blood pH within a narrow range around 7.35.

The activity monitoring unit of the implantable leadless pacemaker of the invention thus responds to both, physical activity and emotional stress. The term "activity" is thus used in broad sense, because it includes both physical activity and emotional stress.

While pH-based rate-adaptive pacemakers using cardiac leads were proposed and tested in the late 70s, they did not gain clinical acceptance as the measurement techniques proposed required an Ag/AgCl reference electrode, which is not biocompatible or suitable for long term implantation.

The leadless pacemaker of the present invention incorporates two electrodes made of biocompatible materials with different sensitivity to pH changes. A first material presents a Nernstian or super-Nernstian behavior with pH, i.e. its open-circuit potential varies with a large negative slope, for example of about less than -50 mV/pH, preferably less than -60 mV/pH, and the second material is either insensitive to pH or presents a smaller negative slope (sub-Nernstian behavior), e.g. a slope of more than -40 mV/pH, preferably more than -30 mV/pH.

In the implantable pacemaker, the open-circuit potential difference between these two electrodes is periodically measured to allow adjusting the pacing rate. The materials of the present invention are also suitable for electrical stimulation, which allows implementing a rate-adaptive pacemaker with the minimum two electrodes required for pacing.

The technical advantages of this invention include: 1) Implementing a rate-adaptive leadless pacemaker re-utilizing features already required for pacemaker operation; 2) implementing a rate-adaptive leadless pacemaker with minimum power consumption required for rate adaptation; 3) reduced component count and size (no need for an accelerometer or temperature sensor) saves space; 4) fast response time compared to a temperature-based sensor; 5) rate adaptation using pH is also responsive to emotional stress, which cannot be achieved with an accelerometer or temperature-based activity sensor.

In a preferred embodiment of the implantable leadless pacemaker, the activity monitoring unit is adapted to periodically determine the open-circuit potential difference between said two electrodes. Preferably, the implantable leadless pacemaker further comprises a control unit that is connected to the activity monitoring unit and that is adapted to adjust a pacing rate in response to said activity signal generated by said activity monitoring unit.

In preferred embodiments of the implantable leadless pacemaker, the sub-Nernstian material is a coating made of or comprising poly(3,4-ethylenedioxythiophene):poly(styrenesulfonate).

In preferred embodiments of the implantable leadless pacemaker, the super-Nernstian material is a coating made of or comprising iridium oxide (IrO).

Preferably, the super-Nernstian material is a coating made of or comprising a non-stoichiometric composition of iridium oxide, namely either IrO(2-x) or IrO(2+x), in particular IrO(1.5).

In preferred embodiments the coating made of or comprising iridium oxide is deposited on the electrode surface using reactive physical vapor deposition (PVD).

A preferred embodiment of the implantable leadless pacemaker has a housing whereupon the two electrodes are arranged and wherein the activity monitoring unit is adapted to periodically determine the open-circuit potential difference between said two electrodes. The preferred implantable leadless pacemaker further comprises a control unit that is connected to the activity sensor and that is adapted to adjust a pacing rate in response to said activity signal generated by said activity monitoring unit.

Preferably, the activity monitoring unit or the control unit or both are configured to generate an activity signal indicating an increase of pacing rate or increasing the pacing rate, respectively, only when a sudden decrease of pH is determined.

It is further preferred when the activity monitoring unit or the control unit or both are configured to determine or to respond to the activity signal during diastole only.

Similarly, it is further preferred when the activity monitoring unit or the control unit or both are configured evaluate the activity signal so as to determine a contact with an inner heart wall and thus to monitor cardiac contraction during systole.

The implantable leadless pacemaker may further comprise impedance determination circuitry and a third electrode made of or comprising iridium oxide, which is connected to said impedance determination circuitry. In such embodiment, in which preferably the first and/or the second electrodes are also connected to the impedance circuitry, the impedance determination circuitry is configured to determine a tissue-electrode capacitance or changes thereof The third electrode preferably has a smaller surface than the first and second electrode.

In preferred embodiments of both, the implantable leadless pacemaker itself is configured to perform long-term monitoring of pH.

The above and other aspects, features and advantages of the present invention will be more apparent from the following more particular description thereof presented in conjunction with the following drawings, wherein:
- Fig. 1: is an external view of a leadless implantable pacemaker;
- Fig. 2: is a schematic block diagram of some internal components of the pacemaker of figure 1; and
- Fig. 3: is a schematic block diagram of some internal components of an alternative embodiment of a pacemaker according to the invention.

The following description is of the best mode presently contemplated for carrying out the invention. This description is not to be taken in a limiting sense, but is made merely for the purpose of describing the general principles of the invention. The scope of the invention should be determined with reference to the claims.

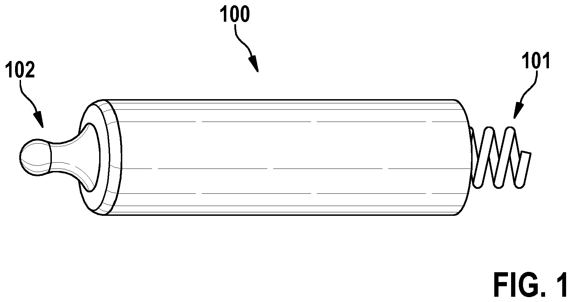

Figure 1 shows an implantable leadless pacemaker with an elongate housing 100 and two electrodes 101 and 102. Each electrode is arranged at a respective longitudinal end of the housing 100.

Electrode 102 is a return electrode for pacing purposes. Electrode 101 is coated with poly(3,4-ethylenedioxythiophene):poly(styrenesulfonate) (also known as PEDOT:PSS), a biocompatible conductive polymer with a linear sub-Nernstian response in the pH range considered. Electrode 102, on the other hand, is coated with iridium oxide deposited with a state of oxidation that provides at least a linear Nernstian behavior with pH.

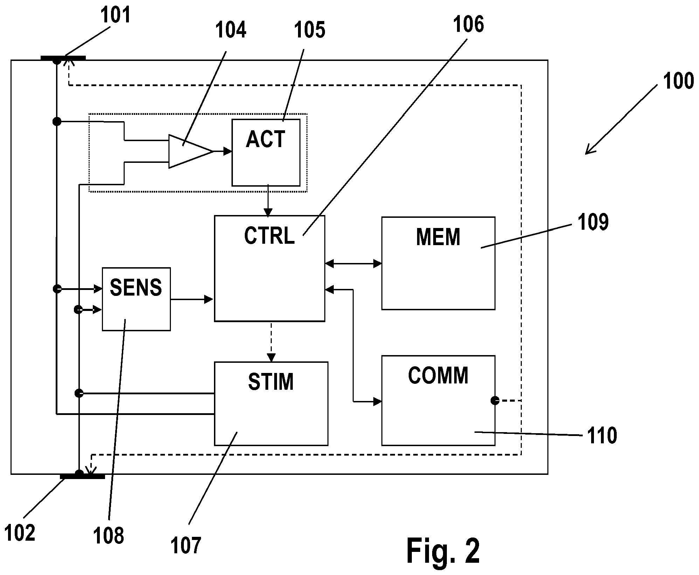

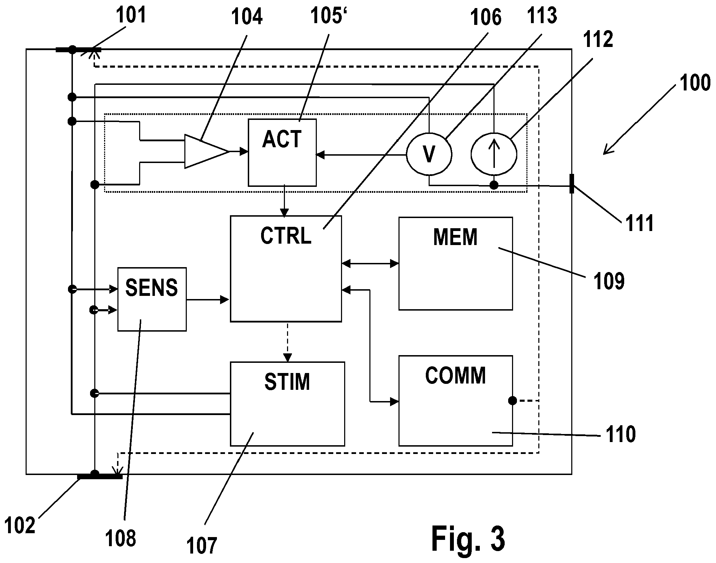

As shown in Figure 2 and Fig. 3, the electrodes 101 and 102 are connected to the inputs of amplifier 104 contained within the housing 100 capable of measuring such varying open-circuit potentials difference. The measured potential is fed to an activity monitoring unit 105 that generates an activity signal for a pacing control unit 106. Pacing control unit 106 is connected to a stimulation unit 107 and a sensing unit 108. Pacing control unit 106 is further connected to a memory 109 and a communication unit 110. By means of stimulation unit 107 and sensing unit 108, control unit 106 can apply demand pacing of a heart chamber through electrodes 101 and 102 in a manner known per se. By means of the activity signal provided by the activity monitoring unit 105, pacing can be rate-adaptive, that is, a respective pacing rate can be adapted to the metabolic demand of a respective person. In another embodiment, the sensing unit 108 is adapted to implement the tasks of 104 thus reducing the circuitry required for rate adaptation. Further, control unit 106 can interact with the electrodes 101 and 102 by means of the communication unit 110.

The implantable leadless pacemaker determines a metabolic demand by means of blood pH changes. Metabolic needs, including emotional stress, will vary the pH in the range of 7.35 to 7.29. Hence, with a resulting slope of -30 mV/pH, this translates into a full-range voltage variation of 1.8 mV. Sixteen steps of rate adaptation imply a resolution of approximately 112 µV, which can be implemented with very low-power consumption.

The two electrodes 101 and 102 are made of biocompatible materials with different sensitivity to pH changes. A first material presents a Nernstian or super-Nernstian behavior with pH, i.e. its open-circuit potential varies with a large negative slope, for example of about - 60 mV/pH, and the second material is either insensitive to pH or presents a smaller negative slope (sub-Nernstian behavior), e.g. a slope of -30 mV/pH.

Since pacing affects the ionic concentrations of the immediate layer next to an electrode, activity monitoring unit 105 and/or control unit 106 are configured to perform measurements for rate adaptation during diastole.

The control unit 106 is configured to increase the pacing rate only when sudden pH decreases are detected. This avoids runaway pacing rate changes that can occur from long-term pH changes in the body due to medication or other systemic effects.

In an alternative embodiment, electrodes 101 and 102 are coated with iridium oxide and PE-DOT:PSS, respectively.

Figure 3 illustrates yet another embodiment, wherein the leadless pacemaker includes a third iridium oxide electrode 111 of much smaller area compared with the other iridium oxide electrode 102. pH variations will result in changes of the tissue-electrode capacitance, which can be detected by impedance measurements or other type of AC measurements. Accordingly, the activity monitoring unit 105' of the embodiment illustrated in Figure 3 further comprises impedance determination circuitry including an AC current source 112 and a voltmeter 113.

In another embodiment, the pH sensor is used for long-term monitoring of blood pH. Changes in blood pH may occur due to medication, and monitoring the pH may help the physician to titrate the proper dosage of medication. pH also may change due to sleep apnea or other metabolic disease states that cause alkalosis or acidosis. The monitor would allow for a physician to monitor the pH trend of the patient and prescribe long-term treatment accordingly.

In yet another embodiment, the mechanical motion of the heart chamber is sensed by measuring the potential of the dissimilar electrode materials in response to contact with the inner heart wall. When the heart wall contacts the electrodes, the thin layer of electrons is perturbed thereby changing the measured potential. The force of the cardiac contraction will alter the potential change on the electrodes. Measurement of the cardiac contraction would occur during systole. Therefore, constant monitoring of the electrode potential during systole and diastole allows for cardiac contractile force measurement as well as venous pH measurements.

For pH sensors, it is preferred to identify materials that undergo a reversible reduction-oxidation reaction when in solution, such that the ratio of activation energies maximizes the resulting cell potential as predicted by the Nernst equation. In the case of iridium oxide, this ratio is facilitated by the transition of Ir from a 4+ to a 3+ charge state (in the form of IrO2 and Ir2O3, respectively). Since the ratio of activation energies is dependent on the molar concentrations of the ions in solutions (and therefore driven by pH), one can see that the voltage sensitivity of the redox reaction can be changed by altering the amount of molar concentration of highly active constituents. In this regard, a preferred iridium oxide coating may actually be a non-stoichiometric composition, meaning that the deposited coating is either IrO(2-x) or IrO(2+x). In this regard, it is possible to tailor the Nernst potential in such a way that it matches specific device and/or sensor requirements.

In a preferred embodiment, the IrOx is deposited on the surface of the device or device component using reactive physical vapor deposition (PVD), although chemical vapor deposition (CVD), or related techniques (PE-CVD, ion beam, etc.) may be used. In reactive PVD, the stoichiometry of the resulting IrOx coating is dependent on the amount of oxygen gas injected into the carrier gas (typically Ar), and the amount of iridium sputtered from a pure target. In this manner, it is possible to create a variety of iridium oxide compositions; the most preferred stoichiometry would be IrO(1.5).

In a preferred embodiment, an IrOx coating is developed such that it exhibits Nernstian or super-Nernstian behavior, and is paired with a counter electrode that provides sub-Nernstian behavior. It is possible that this counter electrode could be a different IrOx composition, as well as PEDOT, bare metal, or other materials.

Although an exemplary embodiment of the present invention has been shown and described, it should be apparent to those of ordinary skill that a number of changes and modifications to the invention may be made without departing from the spirit and scope of the invention. In particular, it is possible to integrate the activity monitor according to the invention in devices other than pacemakers. This invention can readily be adapted to a number of different kinds of medical devices by following the present teachings. All such changes, modifications and alterations should therefore be recognized as falling within the scope of the present invention.

### List of references

- 100: (elongate) housing
- 101, 102: electrodes
- 104: amplifier
- 105, 105': activity monitoring unit
- 106: pacing control unit
- 107: stimulation unit
- 108: sensing unit
- 109: memory
- 110: communication unit
- 111: iridium oxide electrode
- 112: AC current source
- 113: voltmeter

## Claims

1. An implantable leadless pacemaker comprising an activity monitor for monitoring metabolic demand, wherein the monitoring device having at least two electrodes (101, 102) that are made of biocompatible materials that have different sensitivities to pH changes, said two electrodes being connected to an activity monitoring unit (105) that is adapted to periodically determine an open-circuit potential difference between said two electrodes (101, 102) and to generate an activity signal derived from said open-circuit potential difference, said implantable leadless pacemaker having a housing (100), said two electrodes (101, 102) being arranged on said housing (100), and further comprising a control unit (106) that is connected to said activity monitoring unit (105) and that is adapted to adjust a pacing rate in response to said activity signal generated by said activity monitoring unit (105),
**characterized in that**
the activity monitoring unit (105) or the control unit (106) or both are configured to generate an activity signal indicating an increase of pacing rate or an increasing pacing rate, respectively, only when a sudden decrease of pH is determined.

2. The implantable leadless pacemaker of claim 1, wherein one of said two electrodes (101, 102) is made of or comprises a first biocompatible material that presents a Nernstian or super-Nernstian behavior with pH, and the other of said two electrode is made of or comprises a second biocompatible material that is either in-sensitive to pH or presents a sub-Nernstian behavior.

3. The implantable leadless pacemaker of claim 1 or 2, wherein said first biocompatible material that presents a Nernstian or super-Nernstian behavior is a material exhibiting a variation of open-circuit potential with a large negative slope of less than -50 mV/pH, preferably less than -60 mV/pH, and wherein said second biocompatible material, that presents a sub-Nernstian behavior, is a material exhibiting a variation of open-circuit potential with a smaller negative slope of more than -40 mV/pH, preferably more than -30 mV/pH.

4. The implantable leadless pacemaker according to at least one of claims 1 to 3, wherein the sub-Nernstian material is a coating made of or comprising poly(3,4-ethylenedioxythiophene):poly(styrenesulfonate).

5. The implantable leadless pacemaker according to at least one of claims 1 to 4, wherein the super-Nernstian material is a coating made of or comprising iridium oxide.

6. The implantable leadless pacemaker according to claim 5, wherein the super-Nernstian material is a coating made of or comprising a non-stoichiometric composition of iridium oxide, namely either IrO(2-x) or IrO(2+x).

7. The implantable leadless pacemaker according to claim 6, wherein the super-Nernstian material is a coating made of or comprising IrO(1.5) as a non-stoichiometric composition of iridium oxide.

8. The implantable leadless pacemaker according to at least one of claims 5 to 7, wherein the coating made of or comprising iridium oxide is deposited on the electrode surface via reactive physical vapor deposition (PVD).

9. The implantable leadless pacemaker according to claim 1, wherein the activity monitoring unit (105) or the control unit (106) or both are configured to determine or to respond to the activity signal during diastole only.

10. The implantable leadless pacemaker according to at least one of claims 1 or 9, wherein the activity monitoring unit (105) or the control unit (106) or both are configured evaluate the activity signal so as to determine a contact with an inner heart wall and thus to monitor cardiac contraction during systole.

11. The implantable leadless pacemaker according to at least one of claims 1, 9 or 10, further comprising an impedance determination circuitry and a third electrode (111), said third electrode is made of or comprising iridium oxide and is connected to said impedance determination circuitry (112, 113), wherein said impedance determination circuitry (112, 113) is configured to determine a tissue-electrode capacitance or changes thereof.

12. The implantable leadless pacemaker according to claim 11, wherein said third electrode (111) has a smaller surface than said two electrodes (101, 102).

13. The implantable leadless pacemaker according to at least one of claims 1 to 12, further being configured to perform long-term monitoring of pH.

## Patentansprüche

1. Implantierbarer kabelloser Schrittmacher, der eine Aktivitätsüberwachung zum Überwachen des Stoffwechselbedarfs umfasst, wobei die Überwachungsvorrichtung mindestens zwei Elektroden (101, 102) aufweist, die aus biokompatiblen Werkstoffen mit unterschiedlicher Empfindlichkeit gegenüber Änderungen des pH-Werts gefertigt sind, wobei die beiden Elektroden mit einer Aktivitätsüberwachungseinheit (105) verbunden sind, die so ausgelegt ist, dass sie in regelmäßigen Abständen eine Potenzialdifferenz im offenen Stromkreis zwischen den beiden Elektroden (101, 102) ermittelt und ein Aktivitätssignal erzeugt, das aus der Potenzialdifferenz im offenen Stromkreis abgeleitet wird, wobei der implantierbare kabellose Schrittmacher ein Gehäuse (100) aufweist, die beiden Elektroden (101, 102) an dem Gehäuse (100) angeordnet sind und er ferner eine Steuereinheit (106) umfasst, die mit der Aktivitätsüberwachungseinheit (105) verbunden und so ausgelegt ist, dass sie eine Stimulationsfrequenz als Reaktion auf das von der Aktivitätsüberwachungseinheit (105) erzeugte Aktivitätssignal anpasst, **dadurch gekennzeichnet, dass**
die Aktivitätsüberwachungseinheit (105) und/oder die Steuereinheit (106) so ausgelegt sind, dass sie ein Aktivitätssignal, das einen Anstieg der Stimulationsfrequenz beziehungsweise eine zunehmende Stimulationsfrequenz anzeigt, nur dann erzeugen, wenn ein plötzlicher Abfall des pH-Werts ermittelt wird.

2. Implantierbarer kabelloser Schrittmacher nach Anspruch 1, wobei eine der beiden Elektroden (101, 102) aus einem ersten biokompatiblen Werkstoff gefertigt ist oder ihn umfasst, der ein pH-Wert-abhängiges Nernst-Verhalten oder Super-Nernst-Verhalten zeigt, , und die andere der beiden Elektroden aus einem zweiten biokompatiblen Werkstoff gefertigt ist oder ihn umfasst, der entweder unempfindlich gegenüber dem pH-Wert ist oder ein Sub-Nernst-Verhalten zeigt,.

3. Implantierbarer kabelloser Schrittmacher nach Anspruch 1 oder 2, wobei der erste biokompatible Werkstoff, der ein Nernst-Verhalten oder Super-Nernst-Verhalten zeigt, ein Werkstoff mit einer Änderung des Potenzials im offenen Stromkreis mit einer starken negativen Steigung von weniger als -50 mV/pH, vorzugsweise weniger als -60 mV/pH ist, und wobei der zweite biokompatible Werkstoff, der ein Sub-Nernst-Verhalten zeigt, ein Werkstoff mit einer Änderung des Potenzials im offenen Stromkreis mit einer geringeren negativen Steigung von mehr als -40 mV/pH, vorzugsweise mehr als -30 mV/pH ist.

4. Implantierbarer kabelloser Schrittmacher nach mindestens einem der Ansprüche 1 bis 3, wobei der Sub-Nernst-Werkstoff eine Beschichtung ist, die aus Poly(3,4-ethylendioxythiophen)/Poly(styrolsulfonat) gefertigt ist oder Poly(3,4-ethylendioxythiophen)/Poly(styrolsulfonat) umfasst.

5. Implantierbarer kabelloser Schrittmacher nach mindestens einem der Ansprüche 1 bis 4, wobei der Super-Nernst-Werkstoff eine Beschichtung ist, die aus Iridiumoxid gefertigt ist oder Iridiumoxid umfasst.

6. Implantierbarer kabelloser Schrittmacher nach Anspruch 5, wobei der Super-Nernst-Werkstoff eine Beschichtung ist, die aus einer nicht-stöchiometrischen Iridiumoxid-Zusammensetzung gefertigt ist oder eine nicht-stöchiometrische Iridiumoxid-Zusammensetzung umfasst, und zwar entweder IrO(2-x) oder IrO(2+x).

7. Implantierbarer kabelloser Schrittmacher nach Anspruch 6, wobei der Super-Nernst-Werkstoff eine Beschichtung ist, die aus IrO(1,5) als nichtstöchiometrischer Iridiumoxid-Zusammensetzung gefertigt ist oder IrO(1,5) als nicht-stöchiometrische Iridiumoxid-Zusammensetzung umfasst.

8. Implantierbarer kabelloser Schrittmacher nach mindestens einem der Ansprüche 5 bis 7, wobei die Beschichtung, die aus Iridiumoxid gefertigt ist oder Iridiumoxid umfasst, auf der Elektrodenoberfläche mittels reaktiver physikalischer Gasphasenabscheidung (physical vapour deposition, PVD) abgeschieden ist.

9. Implantierbarer kabelloser Schrittmacher nach Anspruch 1, wobei die Aktivitätsüberwachungseinheit (105) und/oder die Steuereinheit (106) so ausgelegt sind, dass sie das Aktivitätssignal nur während der Diastole ermittelt oder darauf reagiert.

10. Implantierbarer kabelloser Schrittmacher nach mindestens einem der Ansprüche 1 oder 9, wobei die Aktivitätsüberwachungseinheit (105) und/oder die Steuereinheit (106) so ausgelegt sind, dass sie das Aktivitätssignal beurteilen und so einen Kontakt mit einer Herzinnenwand ermitteln und so die Herzkontraktion während der Systole überwachen.

11. Implantierbarer kabelloser Schrittmacher nach mindestens einem der Ansprüche 1, 9 oder 10, der ferner eine Impedanzbestimmungsschaltung und eine dritte Elektrode (111) umfasst, wobei die dritte Elektrode aus Iridiumoxid gefertigt ist oder Iridiumoxid umfasst und mit der Impedanzbestimmungsschaltung (112, 113) verbunden ist, wobei die Impedanzbestimmungsschaltung (112, 113) so ausgelegt ist, dass sie eine Gewebe-Elektrode-Kapazität oder Änderungen derselben bestimmt.

12. Implantierbarer kabelloser Schrittmacher nach Anspruch 11, wobei die dritte Elektrode (111) eine kleinere Oberfläche aufweist als die beiden Elektroden (101, 102).

13. Implantierbarer kabelloser Schrittmacher nach mindestens einem der Ansprüche 1 bis 12, der ferner so ausgelegt ist, dass er eine Langzeitüberwachung des pH-Werts vornimmt.

## Revendications

1. Stimulateur cardiaque sans fil implantable comprenant un écran de surveillance de l'activité pour le suivi d'une demande métabolique, où le dispositif de surveillance a au moins deux électrodes (101, 102) qui sont constituées de matériaux biocompatibles qui ont des sensibilités différentes à des changements de pH, lesdites deux électrodes étant reliées à une unité de surveillance de l'activité (105) qui est adaptée pour déterminer périodiquement une différence de potentiel de circuit ouvert entre lesdites deux électrodes (101, 102) et pour générer un signal d'activité dérivé de ladite différence de potentiel de circuit ouvert, ledit stimulateur cardiaque sans fil implantable ayant un boîtier (100), lesdites deux électrodes (101, 102) étant disposées sur ledit boîtier (100), et comprenant en outre une unité de contrôle (106) qui est reliée à ladite unité de surveillance de l'activité (105) et qui est adaptée pour modifier la fréquence de la stimulation en réponse audit signal d'activité généré par ladite unité de surveillance de l'activité (105),
**caractérisé en ce que**
l'unité de surveillance de l'activité (105), ou l'unité de contrôle (106), ou l'ensemble des deux, sont configurées pour générer un signal d'activité indiquant une augmentation de la fréquence de stimulation ou une fréquence de stimulation qui augmente, respectivement, uniquement lorsqu'une diminution soudaine du pH est déterminée.

2. Stimulateur cardiaque sans fil implantable selon la revendication 1, dans lequel l'une desdites deux électrodes (101, 102) est constituée de ou comprend un premier matériau biocompatible qui présente un comportement nernstien ou super nernstien du pH, et l'autre desdites deux électrodes est constituée de ou comprend un second matériau biocompatible qui est soit insensible au pH, soit présente un comportement sub nernstien.

3. Stimulateur cardiaque sans fil implantable selon la revendication 1 ou 2, dans lequel ledit premier matériau biocompatible qui présente un comportement nernstien ou super nernstien est un matériau qui affiche une variation de potentiel du circuit ouvert avec une grande pente négative de moins de -50 mV/pH, de préférence de moins de -60 mV/pH, et où ledit second matériau biocompatible, qui présente un comportement sub nernstien, est un matériau qui affiche une variation de potentiel du circuit ouvert avec une plus petite pente négative de plus de -40 mV/pH, de préférence de plus de -30 mV/pH.

4. Stimulateur cardiaque sans fil implantable selon au moins l'une des revendications 1 à 3, dans lequel la matériau sub nernstien est un revêtement constitué de ou comprenant du poly(3,4-éthylènedioxythiophène) : poly(styrènesulfonate).

5. Stimulateur cardiaque sans fil implantable selon au moins l'une des revendications 1 à 4, dans lequel le matériau super nernstien est un revêtement constitué de ou comprenant de l'oxyde d'iridium.

6. Stimulateur cardiaque sans fil implantable selon la revendication 5, dans lequel le matériau super nernstien est un revêtement constitué de ou comprenant une composition non stoechiométrique d'oxyde d'iridium, à savoir soit IrO(2 - x), soit IrO(2 + x).

7. Stimulateur cardiaque sans fil implantable selon la revendication 6, dans lequel le matériau super nernstien est un revêtement constitué de ou comprenant de I'IrO(1,5) en tant que composition non stoechiométrique de l'oxyde d'iridium.

8. Stimulateur cardiaque sans fil implantable selon au moins l'une des revendications 5 à 7, dans lequel le revêtement constitué de ou comprenant de l'oxyde d'iridium est déposé sur la surface de l'électrode via un dépôt physique réactif en phase vapeur (PVD).

9. Stimulateur cardiaque sans fil implantable selon la revendication 1, dans lequel l'unité de surveillance de l'activité (105), ou l'unité de contrôle (106), ou l'ensemble des deux, sont configurées pour déterminer ou pour répondre au signal d'activité uniquement au cours de la diastole.

10. Stimulateur cardiaque sans fil implantable selon au moins l'une des revendications 1 ou 9, dans lequel l'unité de surveillance de l'activité (105), ou l'unité de contrôle (106), ou l'ensemble des deux, sont configurées pour évaluer le signal d'activité pour déterminer un contact avec une paroi cardiaque interne et surveiller ainsi la contraction cardiaque au cours de la systole.

11. Stimulateur cardiaque sans fil implantable selon au moins l'une des revendications 1, 9 ou 10, comprenant en outre un circuit de détermination de l'impédance et une troisième électrode (111), ladite troisième électrode est constituée de ou comprend de l'oxyde d'iridium et est reliée audit circuit de détermination de l'impédance (112, 113), où ledit circuit de détermination de l'impédance (112, 113) est configuré pour déterminer une capacitance électrode-tissu ou des variations de celle-ci.

12. Stimulateur cardiaque sans fil implantable selon la revendication 11, dans lequel ladite troisième électrode (111) a une surface plus petite que lesdites deux électrodes (101, 102).

13. Stimulateur cardiaque sans fil implantable selon au moins l'une des revendications 1 à 12, étant en outre configuré pour réaliser la surveillance du pH sur le long terme.
